## Europäisches Patentamt
## European Patent Office
## Office européen des brevets

(19)

(11) Veröffentlichungsnummer: **0 185 117**
**A1**

(12) ## EUROPÄISCHE PATENTANMELDUNG

(21) Anmeldenummer: **84116118.5**

(22) Anmeldetag: **21.12.84**

(51) Int. Cl.⁴: **A 61 K 31/49**
**A 23 L 2/02, A 23 L 2/06**
**A 23 L 1/222, A 23 L 1/236**

(43) Veröffentlichungstag der Anmeldung:
**25.06.86 Patentblatt 86/26**

(84) Benannte Vertragsstaaten:
**AT BE CH DE FR GB IT LI LU NL SE**

(71) Anmelder: **IPEX Getränke-Herstellungs- und**
**Vertriebsgesellschaft mbH**
**Obere Binzig 3**
**D-7594 Kappelrodeck(DE)**

(72) Erfinder: **Beck, Klaus**
**Vordere Steige 5**
**D-7800 Freiburg(DE)**

(74) Vertreter: **Vogel, Georg**
**Hermann-Essig-Strasse 35 Postfach 105**
**D-7141 Schwieberdingen(DE)**

(54) **Getränk.**

(57) Getränk, enthaltend Fructose und Vitamin C, zur beschleunigten Senkung des Blutalkoholspiegels sowie Verfahren zur Herstellung des Getränkes. Es ist bekannt, daß Gemische aus Fructose und Vitamin C den Blutalkoholspiegel beschleunigt senken. Die praktische Anwendung dieser Erkenntnis scheiterte bisher an dem unangenehm süßen Geschmack solcher Mischungen. Es wurde nun überraschenderweise gefunden, daß Zusatz von Chinin den widerlich süßen Geschmack eines Fructose und Vitamin C enthaltenden Getränkes völlig verändert und ein leicht bitteres herbes Getränk ergibt, das sehr gut während und nach dem Alkoholgenuß getrunken werden kann. Die vorliegende Erfindung beschreibt die Zusammensetzung eines solchen Getränkes sowie Verfahren zu seiner Herstellung.

Croydon Printing Company Ltd

Getränk

Die vorliegende Erfindung befaßt sich mit einem Getränk, enthaltend Fructose und Vitamin C, zur beschleunigten Senkung des Blutalkoholspiegels, sowie dem Verfahren zur Herstellung des Getränkes.

Seit langem wird nach wirksamen, gut verträglichen und bekömmlichen Mitteln zur Beschleunigung des Blutalkoholabbaus gesucht. Es ist bekannt, daß zum Beispiel die Arzneistoffe Phenobarbitol und Tolbutamid den Alkoholabbau beschleunigen. Der Alkoholtrinker wird jedoch wegen der schwierigen Zugänglichkeit von Arzneimittel und wegen deren möglicher Nebenwirkungen solche Mittel nicht einnehmen. Er wird Mittel bevorzugen, die auf möglichst natürliche und bekömmliche Weise den Alkoholabbau beschleunigen, ohne sein Wohlbefinden zu gefährden. Er bevorzugt außerdem solche Mittel, die geschmacklich zum Alkoholgenuß passen und den Genuß des Alkohols nicht beeinträchtigen.

Durch zahlreiche Untersuchungen ist bekannt, daß die in der Natur vorkommenden Substanzen Fructose (Fruchtzucker) und Vitamin C (Ascorbinsäure) den Alkoholabbau beträchtlich beschleunigen [vgl. dazu z.B. H. Dietl et al, Therapiewoche 27, 9365 (1977), G. Schellmann et al, Blutalkohol 16, 186 (1979)] und den Blutalkoholspiegel senken.

Fructose (Fruchtzucker) ist ein natürlich vorkommendes Monosaccarid. Fructose kann auch synthetisch gewonnen werden, z.B. durch Spaltung von Rohrzucker. Ascorbinsäure (Vitamin

C) ist ein Vitamin, das in vielen Pflanzen (vor allem in Zitrusfrüchten) vorkommt und aus natürlichen Rohstoffen oder auch synthetisch gewonnen werden kann.

Alkohol (Äthanol, Äthylalkohol) wird unter Mitwirkung von Enzymen, vor allem der Enzyme Alkoholdehydrogenase und des Coenzyms Nicotinsäureamidadenindinuclectid (NAD, bzw. NADH) abgebaut zu Kohlendioxyd und Wasser. Fructose bewirkt eine Beschleunigung des Abbaus von Alkohol durch eine Beschleunigung der Reoxidation von NADH im Zellplasma, so daß die Leber mehr von dem alkoholabbauenden Enzym Alkoholdehydrogenase zur Verfügung stellen kann. Auch Vitamin C nimmt an Oxidations- und Reduktionsvorgängen teil und wirkt ebenfalls über eine Steigerung der Alkoholdehydrogenase. Eine alkoholabbauende Wirkung wird nur dann erzielt, wenn beträchtliche Mengen an Fructose, nämlich mehr als 30 g, gegeben werden. Dies bringt große Probleme hinsichtlich der Anwendung mit sich, da Fructose eine im Vergleich zu anderen Zuckern sehr hohe Süßkraft besitzt. Der Alkoholtrinker ist nicht bereit, sich seinen Alkoholgenuß durch die Einnahme eines sehr süßen, fructosehaltigen Getränkes beeinträchtigen zu lassen. Er ist auch nicht bereit, große Mengen der süßen Fructose in fester Form zu sich zu nehmen. Auch wenn das Getränk zusätzlich Vitamin C enthält, bleibt es weiter unangenehm süß. So stellte der Bund gegen Alkohol im Straßenverkehr in einer Pressekonferenz fest, daß so große Mengen an Fruchtzucker und Vitamin zu schlecht schmecken, um vertragen zu werden.

Selbst durch den Zusatz von Zitronensäure sowie durch Aromatisierung, z.B. mit Orangen-, Maracuja- oder Zitronenaroma gelingt es nicht, ein Getränk herzustellen, das sich geschmacklich mit alkoholhaltigen Getränken, z.B. Bier oder Wein, verträgt.

An diesem Problem sind bisher alle Anstrengungen gescheitert, ein alkoholsenkendes, Fructose und Vitamin C enthaltendes Getränk erfolgreich für den Alkoholtrinker zur Verfügung zu stellen.

Überraschenderweise wurde nun gefunden, daß der Zusatz von Chinin den widerlich süßen Geschmack eines Fructose und Vitamin C enthaltenden Getränkes völlig verändert. Das Getränk verliert durch den Zusatz von Chinin gänzlich seine Süße, es erhält einen leicht bitteren, herben Geschmack und kann nun sehr gut während oder nach dem Alkoholgenuß getrunken werden. Der Chiningehalt beträgt dabei pro Liter im allgemeinen 20 mg bis 80 mg, bevorzugt 46 mg bis 55 mg.

Statt Chinin können auch Austauschstoffe für Chinin, z.B. Naringin oder der Extrakt der Enzianschlempe verwendet werden.

Zusätzlich wurde überraschenderweise gefunden, daß die zusätzliche Verwendung von Lemonenextrakt, bzw. Zitronenextrakt oder eines Gemisches von Lemonen- und Zitronenextrakt eine weitere wesentliche Geschmacksverbesserung hervorruft. Wesentlich bleibt jedoch auch bei Verwendung dieser Extrakte der zusätzliche Gehalt von Chinin und/oder dessen Austauschstoffen. Der alleinige Zusatz z.B. von Lemonenextrakt ohne Chinin bewirkt keine befriedigende Überdeckung der Süße eines Fructose und Vitamin C enthaltenden Getränkes.

Der Gehalt an Lemonenextrakt, bzw. Zitronenextrakt beträgt im allgemeinen 8 - 50 g pro Liter, bevorzugt 8 bis 30 g pro Liter.

Lemonenextrakt bzw. Zitronenextrakt enthält bereits Zitronensäure. Zusätzlich kann zur Geschmacksabrundung noch Zitronensäure zugegeben werden. Falls gewünscht, kann das erfindungsgemäße Getränk auch unter Zusatz von Kohlensäure hergestellt werden, wie dies bei Limonadengetränken üblich ist. Dies erhöht seine erfrischende Wirkung.

Der Fructosegehalt und Gehalt an Vitamin C des erfindungsgemäßen Getränkes muß hoch genug sein, um eine den Alkoholabbau fördernde Wirkung zu entfalten.

Der Gehalt an Fructose sollte pro Liter 40 g bis 400 g, bevorzugt 120 bis 320 g, insbesondere 180 bis 290 g betragen.

Der Gehalt an Vitamin C beträgt sinnvollerweise pro Liter 0,4 bis 4 g, bevorzugt 1,5 g bis 3 g.

Das erfindungsgemäße, chininhaltige Getränk aus Fructose und Vitamin C wird durch Mischen der benötigten Substanzen in beliebiger Reihenfolge mit kohlensäurehaltigem oder kohlensäurefreiem Wasser hergestellt. Nach dem Mischen wird mit Wasser (kohlensäurehaltig oder kohlensäurefrei) auf die gewünschte Konzentration eingestellt und das Getränk in Flaschen oder Dosen abgefüllt.

Die verwendete Fructose muß nicht zu hundert Prozent aus reiner Fructose bestehen, aus wirtschaftlichen Gründen kann es angezeigt sein, eine Fructose zu verwenden, die z.B. noch zusätzlich als Verunreinigung Glucose und/oder Saccharose enthält. Fructose kann z.B. als 90 %-iger Fructosesirup eingesetzt werden. Das verwendete Vitamin C kann natürlichen oder synthetischen Ursprungs sein, und z.B. im Lemonenextrakt bereits in der benötigten Menge enthalten sein. Das Chinin kann als reines Chinin zugesetzt oder z.B. bereits dem Lemonenextrakt zugesetzt werden.

Unabhängig von der Art der Herstellung und der Reihenfolge des Mischens ist immer, daß das erfindungsgemäße Getränk Chinin (und/oder dessen Austauschstoffe) in den Mengen enthält, um ein bitter herbes Getränk zu erhalten.

Die folgenden Beispiele erläutern das erfindungsgemäße Getränk weiter, ohne den Bereich der Erfindung einschränken zu wollen.

Beispiel 1:
Durch Mischen der entsprechenden Substanzen mit Wasser wird ein Getränk hergestellt, das pro Liter enthält:

190 g Fructose, 2,5 g Vitamin C, 4 g Citronensäure und 0,8 g natürliches Zitronenaroma.

Das Getränk wurde bei 10 Alkoholtrinkern während des Alkoholgenusses auf seine Akzeptanz geprüft. 9 von 10 Personen weigerten sich, aufgrund des widerlich süßen Geschmackes mehr als 100 ml des Getränkes zu sich zu nehmen.

Es wurde nun ein Getränk gemäß der vorliegenden Erfindung hergestellt durch Mischen der angegebenen Substanzen mit Wasser. Das fertige Getränk enthielt pro Liter 190 g Fructose, 2,5 g Vitamin C, 46 mg Chinin und 19 g Lemonenextrakt. Der herbe, bittere Geschmack wurde nun von allen 10 Alkoholtrinkern voll akzeptiert, da der Geschmack den Alkoholgenuß nicht beeinträchtigte. Alle Personen tranken mindestens 500 ml dieses Getränkes.

Beispiel 2:
Es wurde ein Getränk gemäß der vorliegenden Erfindung hergestellt, indem in 500 l kohlesäurehaltiges Wasser unter Rühren

240 kg Fructosesirup 90 %, 3 kg Vitamin C, 20 kg Lemonenextrakt und 50 g Chinin gemischt wurden. Dann wurde mit kohlensäurehaltigem Wasser auf 1.000 l aufgefüllt und das Getränk in Flaschen zu je 0,33 l abgefüllt.

Das bitter herbschmeckende Getränk wurde von 20 alkoholtrinkenden Personen während des Alkoholgenusses getrunken (ein bis zwei Flaschen pro Person) und als außerordentlich wohlschmeckend und bekömmlich beurteilt.

Beispiel 3:
Einer Gruppe von 10 Personen wurde nach 3-stündigem Fasten pro Person 300 ml Whisky, verdünnt mit 300 ml Wasser, innerhalb von 30 Minuten zum Trinken gegeben.

Innerhalb der nächsten 3 Stunden wurde alle 30 Minuten der Blutalkoholspiegel und dadurch die Abbaurate des Alkohols bestimmt. Die Abbaurate betrug im Mittel 16,5 mg/100 ml/Std.

Nach 3 Stunden nahmen die Personen in einem Zeitraum von 15 Minuten 500 ml eines erfindungsgemäßen, kohlensäurehaltigen Getränkes mit folgender Zusammensetzung zu sich:

| | |
|---|---|
| Fructose | 200 g/l |
| Vitamin C | 3 g/l |
| Chinin | 46 mg/l |
| Lemonenextrakt | 22 g/l |

sonstige Zucker: Glucose 15 g/l, Saccharose 15 g/l.

Alle Personen beurteilten den mild herben Geschmack des Getränkes als ausgezeichnet. Wieder wurde alle 30 Minuten der Blutalkoholspiegel bestimmt und daraus die Geschwindigkeit des Alkoholabbaues berechnet. Der Alkoholabbau war nun im Mittel beträchtlich gesteigert auf 21,1 mg/100 ml/Std.

IPEX Getränke-Herstellungs-
und Vertriebsgesellschaft mbH
Obere Binzig 3

7594  Kappelrodeck

- 1 -

A n s p r ü c h e

1.  Getränk, enthaltend Fructose und Vitamin C, zur
    beschleunigten Senkung des Blutalkoholspiegels,
    dadurch gekennzeichnet,
    daß das Getränk Chinin und/oder deren Austauschstoffe
    enthält.

2.  Getränk nach Anspruch 1,
    dadurch gekennzeichnet,
    daß der Chiningehalt pro Liter 20 mg bis 80 mg, bevorzugt
    46 mg bis 55 mg, beträgt.

3.  Getränk nach Anspruch 1,
    dadurch gekennzeichnet,
    daß der Chininaustauschstoff Naringin oder der Extrakt
    der Enzianschlempe enthalten ist.

4.  Getränk nach den Ansprüchen 1 bis 3,
    dadurch gekennzeichnet,
    daß das Getränk zusätzlich Lemonenextrakt enthält.

5.  Getränk nach Anspruch 4,
    dadurch gekennzeichnet,
    daß das Getränk pro Liter 8 g bis 50 g Lemonenextrakt,
    bevorzugt 12 g bis 30 g Lemonenextrakt, enthält.

6. Getränk nach den Ansprüchen 1 bis 3,

   dadurch gekennzeichnet,

   daß das Getränk zusätzlich Zitronenextrakt enthält.

7. Getränk nach Anspruch 6,

   dadurch gekennzeichnet,

   daß das Getränk pro Liter 8 g bis 50 g Zitronenextrakt, bevorzugt 12 g bis 30 g Zitronenextrakt, enthält.

8. Getränk nach den Ansprüchen 1 bis 3,

   dadurch gekennzeichnet,

   daß das Getränk zusätzlich Lemonenextrakt plus Zitronenextrakt enthält.

9. Getränk nach den Ansprüchen 1 bis 8,

   dadurch gekennzeichnet,

   daß das Getränk Zitronensäure enthält.

10. Getränk nach den Ansprüchen 1 bis 9,

    dadurch gekennzeichnet,

    daß das Getränk pro Liter 3 g bis 30 g Zitronensäure enthält.

11. Getränk nach den Ansprüchen 1 bis 10,

    dadurch gekennzeichnet,

    daß das Getränk Kohlensäure enthält.

12. Getränk nach den Ansprüchen 1 bis 11,

    dadurch gekennzeichnet,

    daß das Getränk pro Liter 40 g bis 400 g Fructose, bevorzugt 120 g bis 320 g Fructose, insbesondere 180 g bis 290 g Fructose und 0,4 g bis 4 g Vitamin C, bevorzugt 1,5 g bis 3 g Vitamin C, enthält.

A 5756        - 3 -        **0185117**

13. Getränk nach den Ansprüchen 1 bis 12,
    dadurch gekennzeichnet,
    daß das Getränk pro Liter 220 - 280 g Fructose, 2 g
    bis 3 g Vitamin C, 20 - 80 mg Chinin, 15 g bis 25 g
    Lemonenextrakt, sowie Kohlensäure enthält.

14. Getränk nach den Ansprüchen 1 bis 13,
    dadurch gekennzeichnet,
    daß das Getränk neben Fructose auch noch andere Zucker,
    z.B. Glucose und Saccharose, enthalten kann.

15. Verfahren zur Herstellung eines Getränkes nach den
    Ansprüchen 1 bis 14,
    dadurch gekennzeichnet,
    daß die Inhaltsstoffe in beliebiger Reihenfolge in
    kohlensäurefreiem oder kohlensäurehaltigem Wasser gelöst
    werden, auf die gewünschte Konzentration mit Waser
    aufgefüllt und das Getränk in Flaschen oder Dosen
    abgefüllt wird.

Europäisches
Patentamt

**EUROPÄISCHER RECHERCHENBERICHT**

0185117

Nummer der Anmeldung

EP 84 11 6118

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | DE-A-3 115 348 (W. LEONHARD)<br>* Ansprüche 1-15 *<br><br>--- | 1-15 | A 61 K 31/49<br>A 23 L 2/02<br>A 23 L 2/06<br>A 23 L 1/222<br>A 23 L 1/236 |
| A | GB-A-2 122 471 (GENERAL FOODS CORP.)<br>* Zusammenfassung *<br><br>----- | | |

RECHERCHIERTE
SACHGEBIETE (Int. Cl.4)

A 61 K 31/00
A 23 L 1/00
A 23 L 2/00

Der vorliegende Recherchenbericht wurde für alle Patentansprüche erstellt.

| Recherchenort<br>BERLIN | Abschlußdatum der Recherche<br>06-08-1985 | Prüfer<br>SCHULTZE D |
|---|---|---|